# EUROPÄISCHE PATENTSCHRIFT

(11) **EP 0 971 907 B1**
(45) Veröffentlichungstag und Bekanntmachung des Hinweises auf die Patenterteilung: **27.02.2002**
(21) Anmeldenummer: 98919095.4
(22) Anmeldetag: 13.03.1998
(51) Int. Cl.: C07D 277/32, C07D 213/61, C07D 405/06, C07D 417/14

(54) **VERFAHREN ZUR HERSTELLUNG VON 1,3-DISUBSTITUIERTEN 2-NITROGUANIDINEN**
METHOD FOR PRODUCING 1,3-DI-SUBSTITUTED 2-NITROGUANIDINES
PROCEDE DE PREPARATION DE 2-NITROGUANIDINES 1,3-DISUBSTITUEES

(30) Priorität: 25.03.1997 DE 19712411
(43) Veröffentlichungstag der Anmeldung: 19.01.2000
(73) Patentinhaber: Bayer Aktiengesellschaft, 51368 Leverkusen (DE)
(72) Erfinder: WOLLWEBER, Detlef, D-42113 Wuppertal (DE); KRÄMER, Wolfgang, D-51399 Burscheid (DE); RIVADENEIRA, Eric, D-51381 Leverkusen (DE)
(86) Internationale Anmeldenummer: EP9801456
(87) Internationale Veröffentlichungsnummer: WO9842690

(56) Entgegenhaltungen:
- EP-A- 0 386 565
- EP-A- 0 483 055
- EP-A- 0 483 062
- CHEMICAL ABSTRACTS, vol. 116, no. 23, 8.Juni 1992 Columbus, Ohio, US; abstract no. 235455, WU F. ET AL: "Preparation of substituted nitroguanidines as insecticides" XP002070585 & JP 03 291 267 A (AGRO-KANESHO CO., LTD.) 20.Dezember 1991 in der Anmeldung erwähnt

## Beschreibung

Die vorliegende Erfindung betrifft ein neuartiges Verfahren zur Herstellung von 1,3-disubstituierten 2-Nitroguanidinen.

Aus EP-A-0 483 062 ist ein Verfahren zur Herstellung von 1,3-disubstituierten 2-Nitroguanidinen bekannt. Sie werden erhalten durch Hydrolyse entsprechender 2-Nitroimino-1,3,5-triazacyclohexanderivate. Die Hydrolyse wird bevorzugt in Gegenwart starker Mineralsäuren oder organischer Säuren durchgeführt.

Nachteilig bei diesem Verfahren sind die langen Reaktionszeiten und das Entstehen von Nebenprodukten, die eine aufwendige Reinigung der gewünschten Endprodukte erforderlich machen.

Darüber hinaus müssen beim Arbeiten in Gegenwart starker Säuren bekanntlich Maßnahmen zum Schutz beispielsweise der Reaktoren gegen Korrosion getroffen werden.

Die JP-03 291 267 betrifft ein ähnliches Verfahren.

Aufgabe der vorliegenden Erfindung war es, ein weiteres Verfahren zur Herstellung von 1,3-disubstituierten 2-Nitroguanidinen bereitzustellen.

Gegenstand der vorliegenden Erfindung ist ein Verfahren zur Herstellung von Verbindungen der Formel (I) 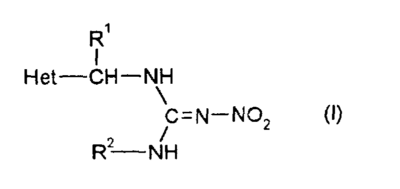 worin
- R¹: für Wasserstoff oder C₁-C₄-Alkyl steht,
- R²: für Wasserstoff, C₁-C₆-Alkyl, C₃-C₆-Cycloalkyl oder einen Rest -CH₂R³ steht,
- R³: für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein oder zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃-Alkoxy, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Halogen, Cyano und Nitro substituiertes 5-Thiazolyl; oder durch ein bis vier (bevorzugt ein oder zwei) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Cyano, Nitro, C₁-C₃-Alkyl, C₁-C₃-Alkoxy und Halogen substituiertes 3-Pyridyl steht,
- Het: für einen unsubstituierten oder substituierten aromatischen oder nichtaromatischen, monocyclischen oder bicyclischen heterocyclischen Rest bevorzugt aus der Reihe 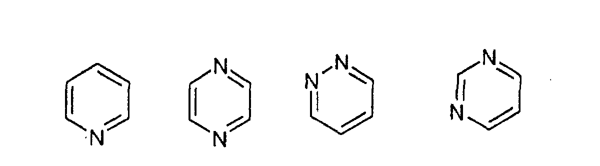 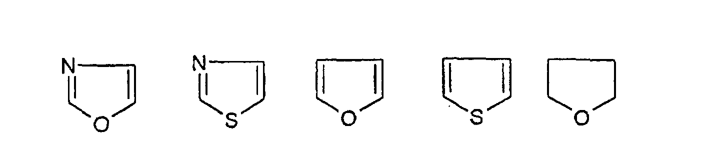 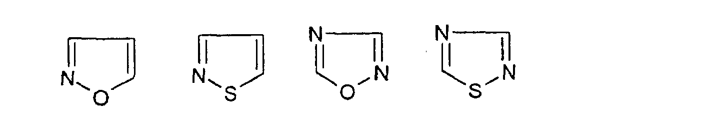 steht, der ein oder zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Cyano, Nitro, C₁-C₃-Alkyl, C₁-C₃-Alkoxy und Halogen enthalten kann,
dadurch gekennzeichnet, daß man eine Verbindung der Formel (II) 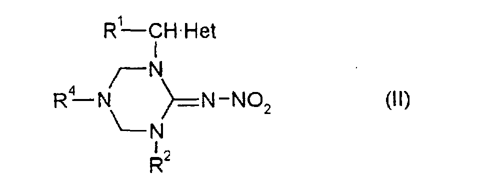 worin
- R¹, R² und Het: die oben angegebenen Bedeutungen haben und
- R⁴: fiir jeweils unsubsituiertes oder substituiertes C₁-C₁₀-Alkyl, C₃-C₆-Cycloalkyl, Phenyl, Benzyl oder Heterocyclylmethyl steht, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht,
in Gegenwart eines Verdünnungsmittels mit Harnstoff umsetzt.

Die Verbindungen der Formel (I) können auch als Doppelbindungsisomere bezüglich der -N=C(2)-Bindung und in ihren tautomeren Formen (Formeln Ia, Ib) auftreten: 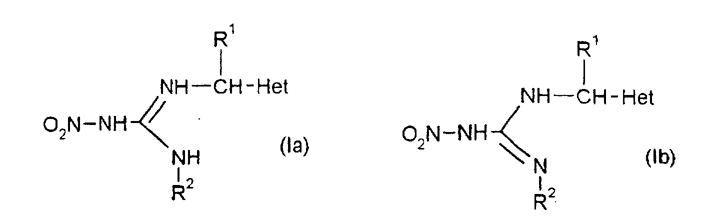 Formel (I) ist demnach so zu verstehen, daß sie auch die entsprechenden Doppelbindungsisomeren und die Formeln (Ia) und (Ib) einschließt.

Überraschenderweise liefert das erfindungsgemäße Verfahren selektiv und in hohen Ausbeuten die Endprodukte der Formel (I) nach kurzer Reaktionszeit unter milden Reaktionsbedingungen in reiner Form.

Verwendet man beispielsweise 1-(2-Chlorthiazol-5-ylmethyl)-2-nitro-imino-3,5-dimethyl-1,3,5-triazacyclohexan als Ausgangsstoff, so kann der Verlauf des erfindungsgemäßen Verfahrens durch das folgende Formelschema wiedergegeben werden:

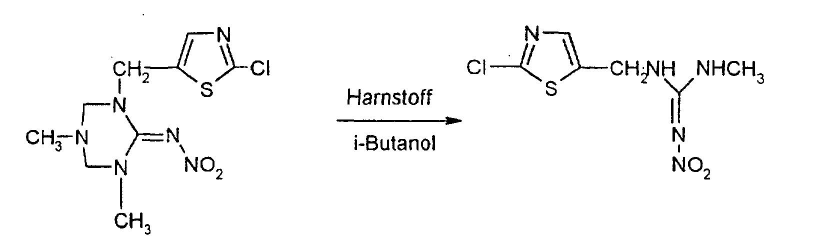

Die als Ausgangsstoffe für das erfindungsgemäße Verfahren benötigten Verbindungen sind durch die Formel (II) allgemein definiert.

Bevorzugte Substituenten bzw. Bereiche der in den oben und nachstehend erwähnten Formeln aufgeführten Reste werden im folgenden erläutert:
- R¹: steht bevorzugt für Wasserstoff, Methyl, Ethyl, n- oder i-Propyl,
- R²: steht bevorzugt für Wasserstoff, Methyl, Ethyl, n-Propyl, i-Propyl oder n-Butyl, Cyclopropyl, Cyclopentyl, Cyclohexyl oder für einen Rest -CH₂R³,
- R³: steht bevorzugt für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein oder zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃-Alkoxy, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Halogen, Cyan und Nitro substituiertes 5-Thiazolyl; oder durch ein oder zwei (bevorzugt einen) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Cyano, Nitro, C₁-C₃-Alkyl, C₁-C₃-Alkoxy und Halogen substituiertes 3-Pyridyl,
- R⁴: steht bevorzugt für C₁-C₁₀-Alkyl, C₃-C₆-Cycloalkyl, durch 1 bis 12 Reste aus der Gruppe Halogen, Hydroxy, C₁-C₄-Alkoxy, C₁-C₄-Halogenalkoxy mit 1 bis 9 Halogenatomen, Di-( C₁-C₄-Alkyl)-amino und C₁-C₅-Alkoxycarbonyl substituiertes C₁-C₁₀-Alkyl, durch 1 bis 4 Reste aus der Reihe C₁-C₄-Alkyl und Halogen substituiertes C₃-C₆-Cycloalkyl, Phenyl, Benzyl, oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro und Cyano substituiertes Phenyl oder Benzyl oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht,
- Het: steht bevorzugt für einen unsubstituierten oder einfach oder zweifach (bevorzugt einfach) substituierten heterocyclischen Rest aus der Reihe 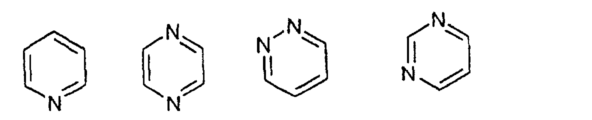 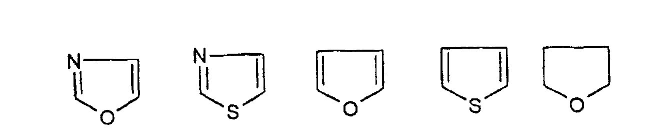 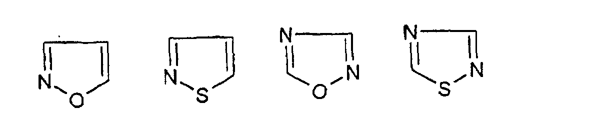 wobei die Substituenten aus der Reihe Fluor, Chlor, Brom, Methyl, Ethyl, Methoxy und Ethoxy ausgewählt sind;
- R¹: steht besonders bevorzugt für Wasserstoff oder Methyl,
- R²: steht besonders bevorzugt für Wasserstoff, Methyl, Ethyl, n-Propyl, i-Propyl, n-Butyl, Cyclopropyl, Cyclopentyl, Cyclohexyl oder für einen Rest -CH₂R³,
- R³: steht besonders bevorzugt für C₂-C₃-Alkenyl, C₂-C₃-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano und Nitro substituiertes 5-Thiazolyl oder 3-Pyridyl,
- R⁴: steht besonders bevorzugt für C₁-C₁₀-Alkyl, C₃-C₆-Cycloalkyl, durch 1 bis 6 Reste aus der Gruppe Halogen, C₁-C₄-Alkoxy und C₁-C₄-Halogenalkoxy mit jeweils 1 bis 9 Halogenatomen substituiertes C₁-C₈-Alkyl, durch 1 oder 2 Reste aus der Reihe Methyl, Ethyl, Fluor oder Chlor substituiertes C₃-C₆-Cycloalkyl, Phenyl, Benzyl, oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro und Cyano substituiertes Phenyl oder Benzyl, oder Heterocyclylmethyl, wobei Heterocyclyl für einen ungesättigten oder gesättigten 5- oder 6-gliedrigen Heterocyclus mit einem Heteroatom aus der Reihe Stickstoff, Sauerstoff und Schwefel wie z.B. Furan, Tetrahydrofuran, Thiophen oder Pyridin steht,
- Het: steht besonders bevorzugt für jeweils unsubstituiertes oder einfach oder zweifach (insbesondere einfach) substituiertes Thiazolyl, Pyridyl oder Tetrahydrofuranyl, wobei die Substituenten aus der Reihe Fluor, Chlor, Methyl und Methoxy (insbesondere Chlor) ausgewählt sind,

Halogen(atome) steht bevorzugt und besonders bevorzugt für Fluor(atome), Chlor(atome) und Brom(atome),
- R¹: steht ganz besonders bevorzugt für Wasserstoff oder Methyl, hervorgehoben für Wasserstoff,
- R²: steht ganz besonders bevorzugt für Wasserstoff, Methyl, Ethyl, n-Propyl, Cyclopropyl, Cyclopentyl, Allyl oder Propargyl,
- R⁴: steht ganz besonders bevorzugt für Methyl, Ethyl, n-Propyl, Cyclopropyl, Cyclopentyl, Cyclohexyl, Phenyl, Benzyl oder Furfurylmethyl,
- Het: steht ganz besonders bevorzugt für einen der Reste

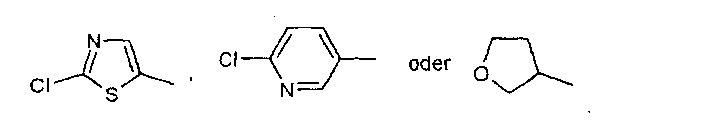

Besonders hervorgehoben seien als Ausgangsstoffe für das erfindungsgemäße Verfahren Verbindungen der Formel (IIa) 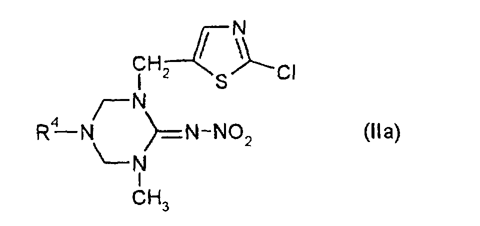 worin
- R⁴: für Methyl, Ethyl, Cyclopropyl, Cyclopentyl, Benzyl oder Furfurylmethyl steht, wobei unter diesen wiederum Methyl, Benzyl und Furfurylmethyl bevorzugt sind.

Besonders hervorgehoben seien als Ausgangsstoffe für das erfindungsgemäße Verfahren Verbindungen der Formel (IIb) und (IIc) 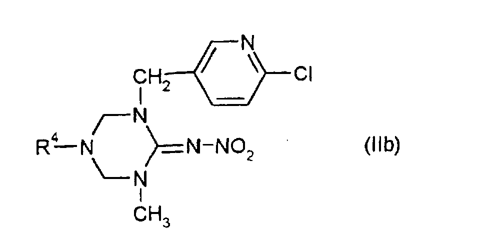 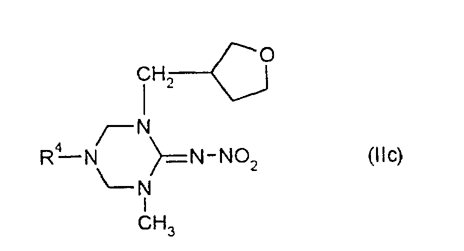 worin
- R⁴: die oben für die Verbindungen der Formel (IIa) angegebenen Bedeutungen hat.

Als Endprodukte des erfindungsgemäßen Verfahrens erhält man bei Verwendung von Verbindungen der Formel (IIa) die folgende Verbindung 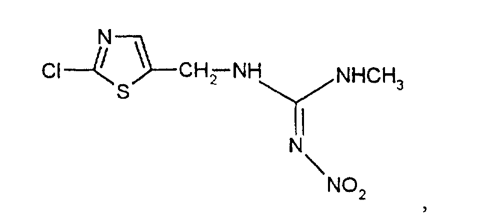 bei Verwendung der Verbindung der Formel (IIb) die folgende Verbindung 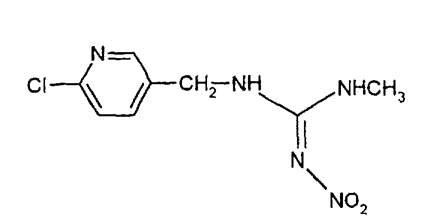 und bei Verwendung der Verbindung der Formel (IIc) die folgende Verbindung

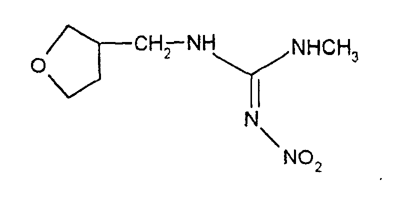

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Bevorzugt werden diejenigen Verbindungen der Formel (II) in das erfindungsgemäße Verfahren eingesetzt, in welchen eine Kombination der vorstehend als bevorzugt aufgeführten Bedeutungen vorliegt.

Besonders bevorzugt werden diejenigen Verbindungen der Formel (II) in das erfindungsgemäße Verfahren eingesetzt, in welchen eine Kombination der vorstehend als besonders bevorzugt aufgeführten Bedeutungen vorliegt.

Ganz besonders bevorzugt werden diejenigen Verbindungen der Formel (II) in das erfindungsgemäße Verfahren eingesetzt, in welchen eine Kombination der vorstehend als ganz besonders bevorzugt aufgeführten Bedeutungen vorliegt.

Die Ausgangsstoffe der Formel (II) sind bekannt oder können nach bekannten Verfahren hergestellt werden (vgl. EP-A-0 483 062, JP-03 291 267, EP-A-0 483 055, EP-A-0 428 941, EP-A-0 386 565).

Man erhält die Verbindungen der Formel (II) beispielsweise, wenn man
a) eine Verbindung der Formel (III) 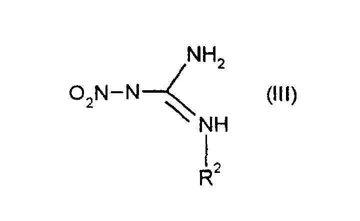 mit Formaldehyd und einer Verbindung der Formel (IV)

   H₂N-R⁴ (IV)

   umsetzt; und
b) die erhaltene Verbindung der Formel (V) 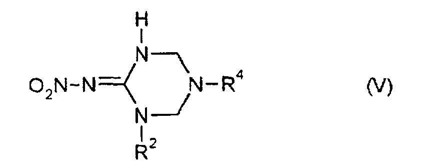 mit einer Verbindung der Formel (VI) 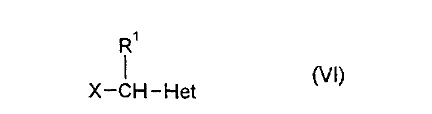 umsetzt, wobei in den Formeln (II) bis (VI) die Reste R¹, R², R⁴ und Het die oben angegebenen Bedeutungen haben und X für eine Abgangsgruppe steht.

Als Abgangsgruppe X können z.B. in Betracht kommen: Halogen, vorzugsweise Chlor, Brom oder Jod, oder Sulfonsäurereste, wie Alkylsulfonsäurereste, beispielsweise Mesylat oder Tosylat.

Die Stufe a) des obigen Verfahrens zur Herstellung der Verbindungen der Formel (II) wird mit Vorteil unter normalem Druck, gegebenenfalls auch unter erhöhtem Druck in einem inerten Lösungsmittel und bei Temperaturen zwischen 0°C und +140°C, vorzugsweise zwischen +20°C und +120°C durchgeführt. Als Lösungsmittel eignen sich in besonderer Weise Alkohole, wie Methanol, Ethanol und Propanol, sowie Wasser. Weitere geeignete Lösungsmittel sind z.B. aromatische Kohlenwasserstoffe, wie Benzol, Toluol und Xylol; Ether wie Tetrahydrofuran, Dioxan und Diethylether; halogenierte Kohlenwasserstoffe, wie Methylenchlorid, Chloroform, Tetrachlorkohlenstoff und Chlorbenzol sowie andere Lösungsmittel, die die Reaktion nicht beeinträchtigen. Die Lösungsmittel können auch als Gemische verwendet werden. Die Verfahrensstufe b) kann vorzugsweise unter normalem oder leicht erhöhtem Druck und in Gegenwart von vorzugsweise aprotischen Lösungs- oder Verdünnungsmitteln durchgeführt werden. Als Lösungs- oder Verdünnungsmittel eignen sich z.B. Ether und etherartige Verbindungen, wie Diethylether, Dipropylether, Dibutylether, Dioxan, Dimethoxyether und Tetrahydrofuran; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol, Nitrile, wie Acetonitril oder Propionitril; Dimethylsulfoxid oder Dimethylformamid sowie Gemische dieser Lösungsmittel. Diese Verfahrensstufe wird im allgemeinen bei einer Temperatur von -20°C bis +140°C, vorzugsweise zwischen 0°C und +120°C, vorzugsweise in Gegenwart einer Base, durchgeführt. Als Basen eignen sich z.B. Carbonate, wie Natrium- und Kaliumcarbonat. Auch Hydride, wie Natriumhydrid, Kaliumhydrid und Calciumhydrid, können als Basen eingesetzt werden. Gegebenenfalls kann die Umsetzung auch in Gegenwart eines Katalysators, z.B. Cäsiumchlorid, durchgeführt werden.

Die Ausgangsprodukte der obigen Formeln (IV) und (VI) sind bekannt und im Handel erhältlich oder können leicht in Analogie zu bekannten Verfahren hergestellt werden. Die 2-Nitroguanidin-Ausgangsprodukte der Formel (III) sind ebenfalls bekannt; sie lassen sich vorteilhaft aus S-Methyl-N-nitroisothioharnstoff durch Umsetzung mit einem entsprechenden primären Amin herstellen (vgl. US-PS 4 804 780 und 4 221 802). N-Methyl-N'-nitroguanidin ist auch durch Umsetzung von Nitroguanidin mit Methylamin erhältlich (vgl. EP-A-0 798 293 der Anmelderin). S-Methyl-N-nitroisothioharnstoff erhält man in guter Ausbeute durch Nitrierung von S-Methylisothioharnstoff(vgl. J. Am. Soc. 76, 1877 (1954)).

Das erfindungsgemäße Verfahren wird in Gegenwart eines Verdünnungsmittels durchgeführt.

Als Verdünnungsmittel eignen sich insbesondere organische Lösungsmittel, besonders polare protische Lösungsmittel, beispielsweise Alkohole wie Methanol, Ethanol, n-Propanol, i-Propanol, n-Butanol oder i-Butanol und polare aprotische Lösungsmittel, beispielsweise Amide wie Dimethylformamid, Dimethylacetamid oder N-Methylpyrrolidon oder Sulfoxide wie Dimethylsulfoxid.

Es ist auch möglich, Gemische der genannten Verdünnungsmittel einzusetzen. Besonders als Verdünnungsmittel geeignet sind Alkohole, insbesondere die oben genannten.

Es kann von Vorteil sein, der Reaktionsmischung ein weiteres Lösungsmittel zuzusetzen. Als solches kommen Ether in Frage, beispielsweise Dibutylether, Tetrahydrofuran, Dioxan, Glykoldimethylether oder Diglykoldimethylether, weiterhin Kohlenwasserstoffe, wie Benzol, Toluol oder Xylol, halogenierte Kohlenwasserstoffe, wie Methylenchlorid, Chloroform, Tetrachlorkohlenstoff, Chlorbenzol oder o-Dichlorbenzol, Nitrile wie Acetonitril, Carbonsäureester wie Ethylacetat oder auch Ketone wie Aceton oder Methylisopropylketon.

Es ist auch möglich, Gemische der genannten Lösungsmittel einzusetzen. Bevorzugt werden Ether verwendet.

Das erfindungsgemäße Verfahren wird bei Temperaturen zwischen 0°C und 200°C, vorzugsweise zwischen 40°C und 150°C, durchgeführt.

Vorzugsweise wird unter Normaldruck gearbeitet.

Der Harnstoff wird im allgemeinen in einem molaren Verhältnis von 1:10 bis 10:1, vorzugsweise 1:4 bis 4:1, insbesondere 1:1 bis 3:1, bezogen auf die Ausgangsverbindung der Formel (II), eingesetzt.

Im allgemeinen wird die Umsetzung so durchgeführt, daß man den Ausgangsstoff der Formel (II) und Harnstoff in dem Verdünnungsmittel und gegebenenfalls im Lösungsmittel auf die gewünschte Temperatur erhitzt.

Zur Aufarbeitung wird nach dem Abkühlen mit Wasser versetzt und das Endprodukt gegebenenfalls nach Einengen des Gemischs beispielsweise durch Filtration oder Extraktion isoliert.

Es ist auch möglich, eine wasserfreie Aufarbeitung des Reaktionsgemisches durchzuführen, indem man nach beendeter Reaktion das Verdünnungsmittel und gegebenenfalls Lösungsmittel abdestilliert und den verbleibenden Rückstand mit einem geeigneten Extraktionsmittel, extrahiert. Als Extraktionsmittel kommen grundsätzlich alle gegenüber den Endprodukten inerten Lösungsmittel in Frage, in denen die Endprodukte ausreichend löslich sind.

Hierzu gehören beispielsweise aliphatische Kohlenwasserstoffe wie n-Pentan, n-Hexan, Cyclohexan, halogenierte aliphatische Kohlenwasserstoffe wie Methylenchlorid oder Chloroform, aromatische Kohlenwasserstoffe wie Benzol, Toluol oder Xylol, halogenierte aromatische Kohlenwasserstoffe wie Chlorbenzol oder o-Dichlorbenzol und auch Ether wie beispielsweise Methyl-tert-butylether.

Die Endprodukte kristallisieren, gegebenenfalls nach Einengen des Extraktionsmittels aus und können durch Filtration isoliert werden oder das Extraktionsmittel wird vollständig oder nahezu vollständig entfernt und der Rückstand, falls nötig, beispielsweise durch Umkristallisation gereinigt.

Bevorzugt wird die Reaktion in einem Verdünnungsmittel durchgeführt, aus welchem beim Abkühlen der Reaktionsmischung das Endprodukt auskristallisiert und in einfacher Weise beispielsweise durch Filtration isoliert werden kann. Hierfür kommen Alkohole in Frage, insbesondere Isobutanol.

Die erfindungsgemäß hergestellten Verbindungen der Formel (I) sind wertvolle Wirkstoffe in der Schädlingsbekämpfung. Insbesondere sind die Verbindungen der Formel (I) geeignet zur Bekämpfung von Insekten und Spinnentieren, die in Nutzund Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen (siehe z.B. EP-A-0 376 279, EP-A-0 375 907, EP-A-0 383 091).

### Beispiel 1

Herstellung von 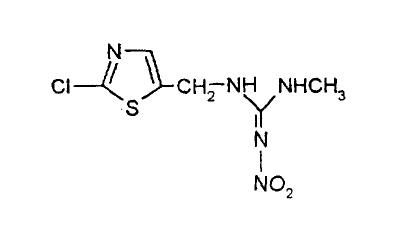 1-(2-Chlorthiazol-5-ylmethyl)-2-nitro-3-methylguanidin
a) 3 g l-(2-Chlorthiazol-5-ylmethyl)-2-nitroimino-3,5-dimethyl-1,3,5-triazacyclohexan der Formel (Herstellung siehe EP-A-0 428 941) 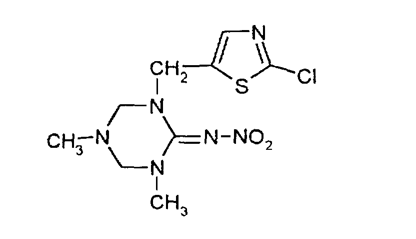 und 1,2 g Harnstoff(H₂N-CO-NH₂) werden in 30 ml Dimethoxyethan suspendiert und nach Zugabe von 10 ml Isobutanol 9 Stunden unter Rückfluß gekocht. Man gibt auf verdünnte wäßrige Natriumchloridlösung, engt die Suspension im Vakuum etwas ein, saugt den Feststoff ab, wäscht mit Wasser und trocknet.
   Ausbeute: 2,0 g HPLC-Reinheit >98 %.
   ¹H-NMR (DMSO-d₆), δ = 2.8 ppm (3H, s); 4.5 ppm (2H, s), 7.6 ppm (1H, s),
   7.9-8.1 ppm (1H, breit), 9.1-9.3 ppm (1H, breit).
   Das Produkt ist nach seinen chromatographischen und spektroskopischen Daten identisch mit einer auf anderem Weg erhaltenen, authentischen Probe 1-(2-Chlorthiazol-5-ylmethyl)-2-nitro-3-methylguanidin.
b) 3 g der gleichen Ausgangsverbindung wie in Beispiel 1a) und 1,2 g Harnstoff werden in 30 ml Isobutanol 6 Stunden unter Rückfluß erhitzt. Nach dem Abkühlen gibt man 200 ml Wasser zu und destilliert das Isobutanol im Vakuum azeotrop ab. Der ausgefallene Feststoff wird abgesaugt und getrocknet. Ausbeute: 2,1 g (86 % der Theorie).
   Analytische Daten wie bei 1a); HPLC-Reinheit >99 %.
c) 15 g der Ausgangsverbindung wie in Beispiel 1a) und 6,0 g Harnstoff werden in 100 ml Isobutanol 8 Stunden unter Rückfluß erhitzt. Man läßt abkühlen und filtriert den gebildeten Feststoff ab, wäscht mit wenig Isobutanol nach und trocknet.
   Ausbeute: 10,2 g (84 % der Theorie). Analytische Reinheit wie bei 1b).
d) 1 g 1-(2-Chlorthiazol-5-ylmethyl)-2-nitroimino-(3-methyl-5-benzyl)-1,3,5-triazacyclohexan (Herstellung EP-A-0 483 055) und 0,3 g Harnstoff werden in 10 ml Isobutanol 10 Stunden auf Rückflußtemperatur erhitzt. Nach Aufarbeitung wie unter b) erhält man 0,4 g der obengenannten Verbindung.
e) 135 kg 1-(2-Chlorthiazol-5-ylmethyl)-2-nitroimino-3-methyl-5-benzyl-1,3,5-triazacyclohexan der Formel 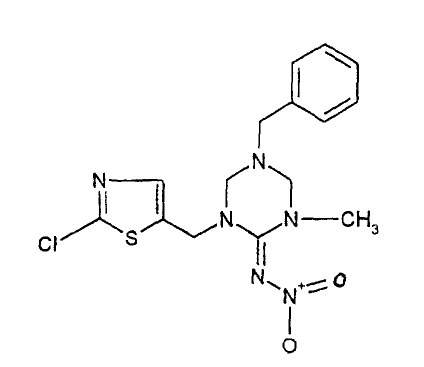 und 53 kg Harnstoff werden in 585 kg Isobutanol 12 Stunden lang auf 100°C erhitzt. Es wird auf 20°C abgekühlt, kristallisiert und durch Filtration isoliert. Das Kristallisat wird mit Wasser gewaschen und anschließend getrocknet. Man erhält 75 kg 1-(2-Chlorthiazol-5-ylmethyl)-2-nitro-3-methylguanidin.

Analog können auch die in der folgenden Tabelle aufgeführten Verbindungen der Formel (I) erhalten werden:

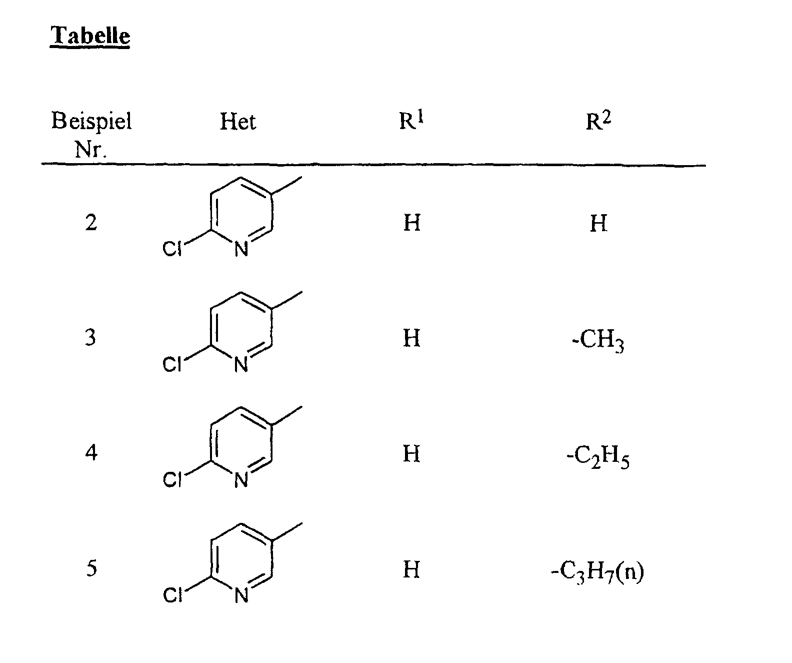

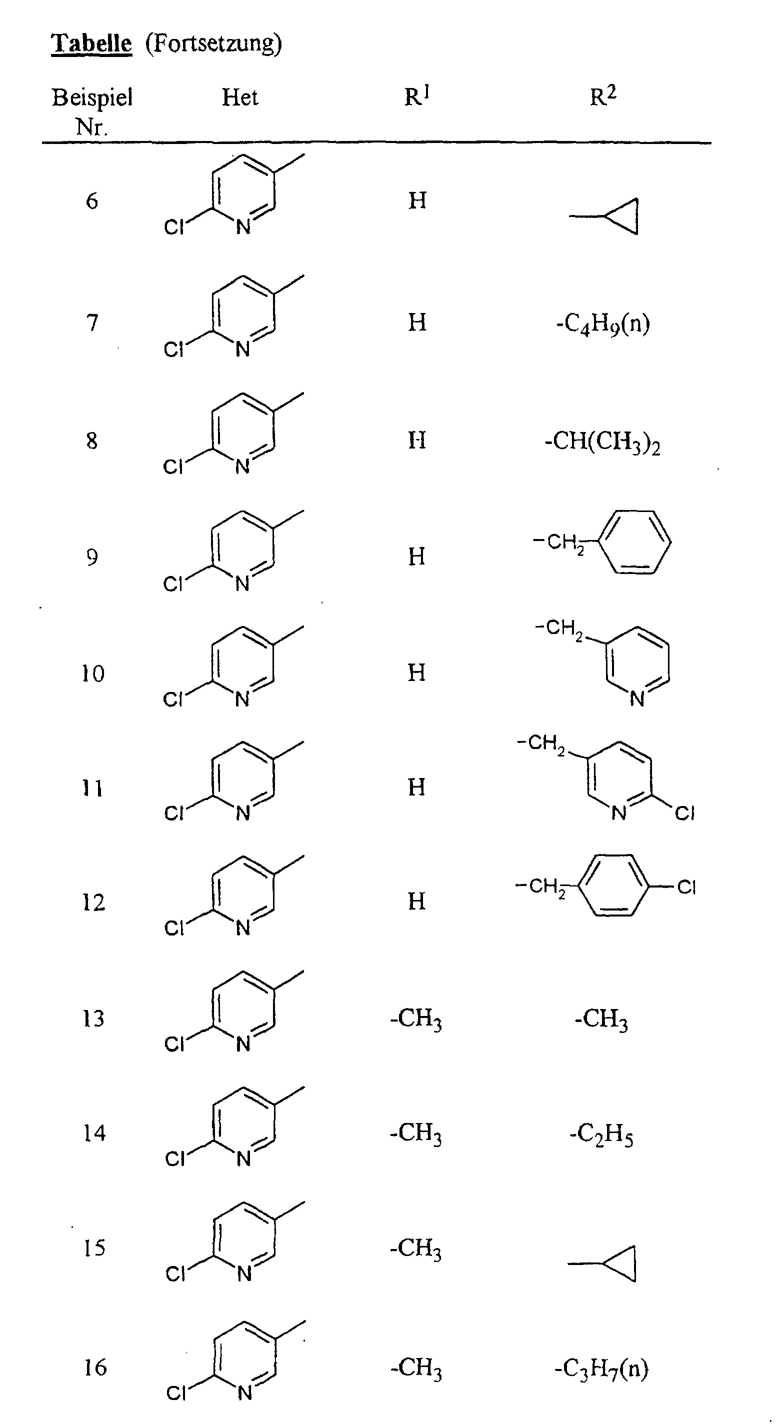

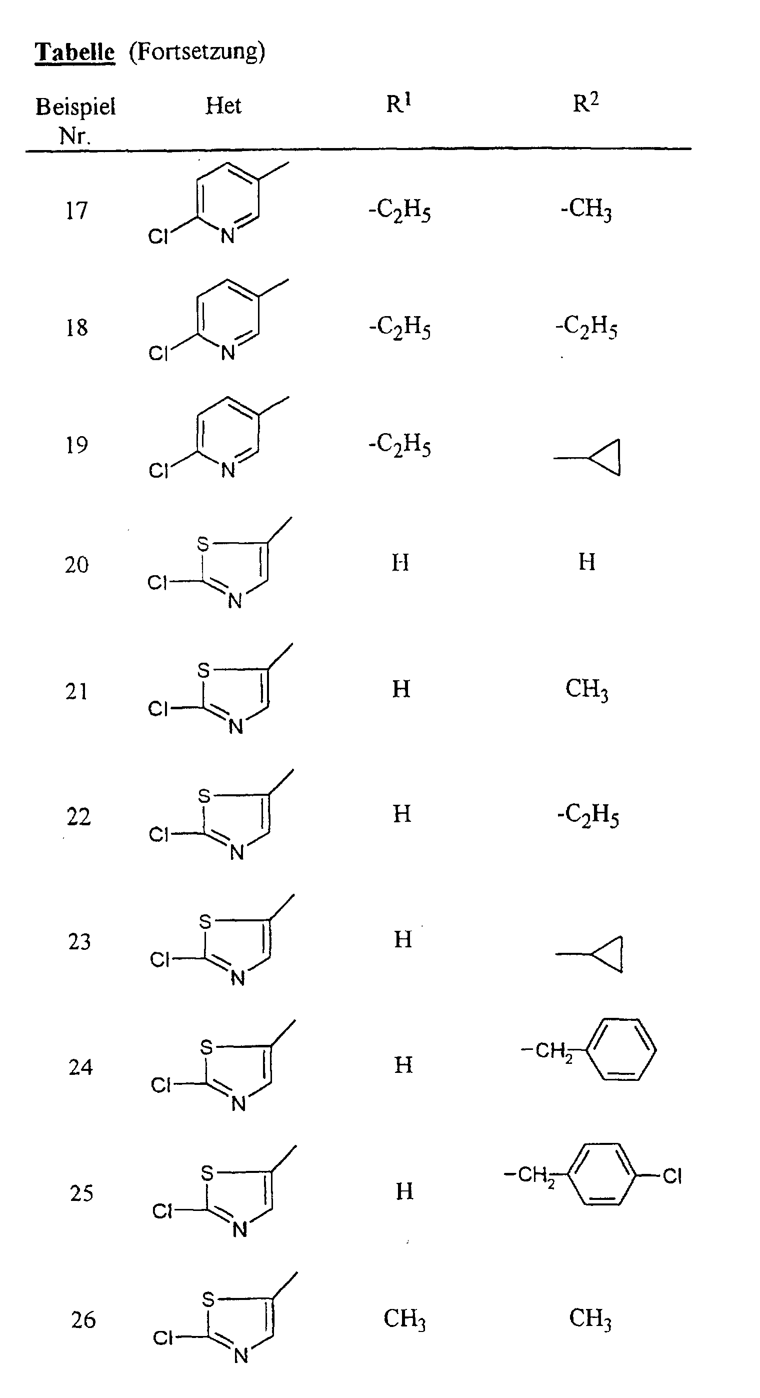

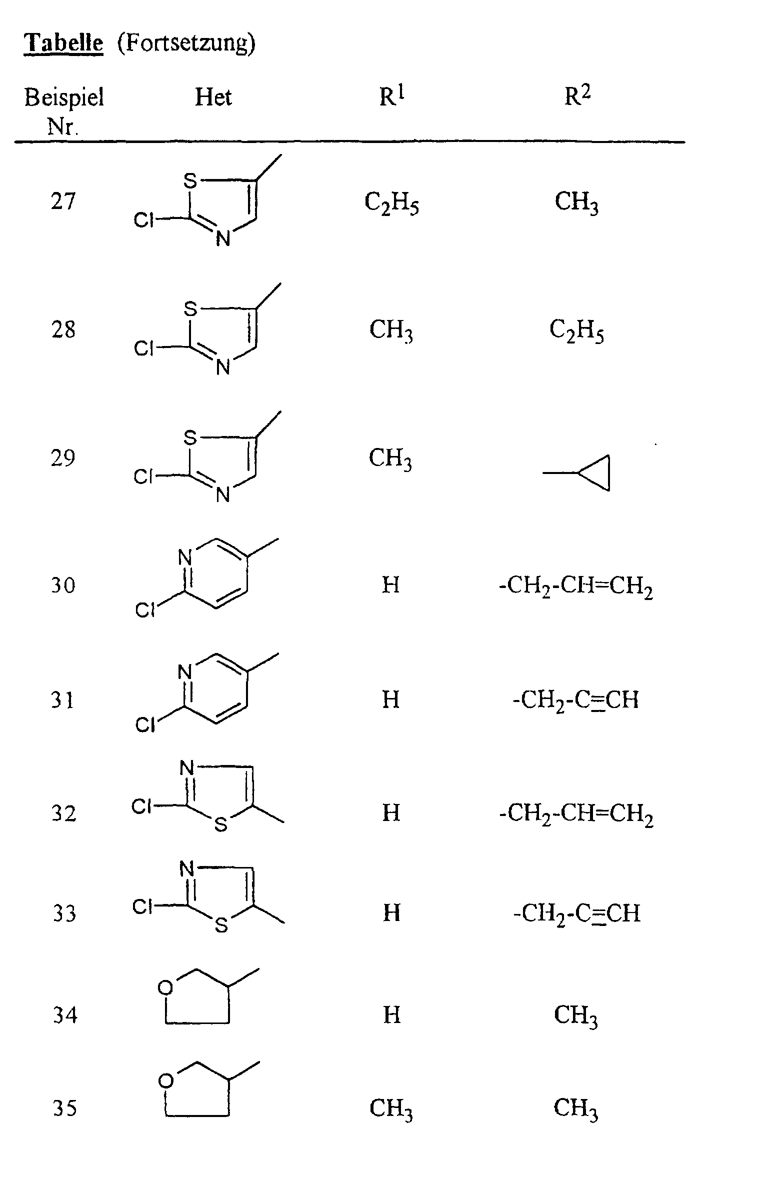

### Herstellung von neuen Ausgangsverbindungen:

### Beispiel (V-1)

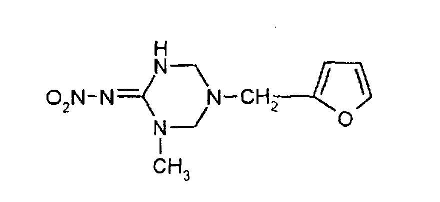

Ein Gemisch aus N-Methylnitroguanidin (98,3 g, 0,5 mol, Wassergehalt 40 %) und 37 %iger wäßriger Formaldehydlösung (200 ml, 2,7 mol) wird 1 Stunde bei 70°C gerührt. Dann wird bei dieser Temperatur eine Lösung von Furfurylamin (51 g, 0,53 mol) in Wasser (50 g) zugegeben. Man rührt nach 2 Stunden bei 70°C und kühlt dann auf 30°C. Bei dieser Temperatur gibt man einige Impfkristalle zu, kühlt auf 20°C, saugt den Niederschlag ab, wäscht mit Wasser und trocknet.
Ausbeute: 104 g (87 % der Theorie).

### Beispiel (II-1)

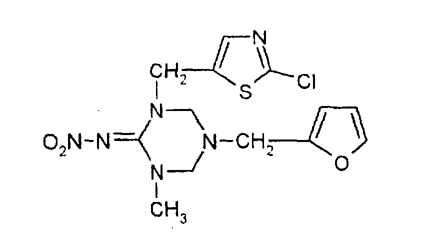

Zum Gemisch von 48 g (0,2 mol) der Verbindung gemäß Beispiel (V-1), 36 g (0,26 mol) gemahlenem Kaliumcarbonat und 100 ml Dimethylformamid gibt man bei 50°C 33,6 g (0,2 mol) 2-Chlor-5-chlormethylthiazol und rührt 16 Stunden bei dieser Temperatur. Man läßt abkühlen, gibt auf Wasser, stellt mit Schwefelsäure etwa neutral, saugt ab, wäscht mit Wasser und trocknet.

## Patentansprüche

1. Verfahren zur Herstellung von Verbindungen der Formel (I) 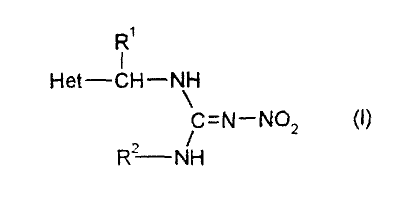 worin
R¹ für Wasserstoff oder C₁-C₄-Alkyl steht,
R² für Wasserstoff, C₁-C₆-Alkyl, C₃-C₆-Cycloalkyl oder einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein oder zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃-Alkoxy, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Halogen, Cyano und Nitro substituiertes 5-Thiazolyl; oder durch ein bis vier (bevorzugt ein oder zwei) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Cyano, Nitro, C₁-C₃-Alkyl, C₁-C₃-Alkoxy und Halogen substituiertes 3-Pyridyl steht,
Het für einen unsubstituierten oder substituierten aromatischen oder nichtaromatischen, monocyclischen oder bicyclischen heterocyclischen Rest steht, der ein bis zwei Substituenten aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy, Halogenallylthio, Cyano, Nitro, C₁-C₃-Alkyl, C₁-C₃-Alkoxy und Halogen enthalten kann,
**dadurch gekennzeichnet, daß** man eine Verbindung der Formel (II) 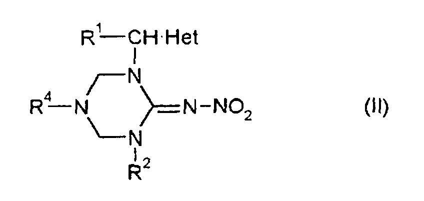 worin
R¹, R² und Het die oben angegebenen Bedeutungen haben und
R⁴ für jeweils unsubsituiertes oder substituiertes C₁-C₁₀-Alkyl, C₃-C₆-Cycloalkyl, Phenyl, Benzyl oder Heterocyclylmethyl steht, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht,
in Gegenwart eines Verdünnungsmittels mit Harnstoff umsetzt.

2. Verfahren gemäß Anspruch 1, **dadurch gekennzeichnet, daß** man eine Verbindung der Formel (II) gemäß Anspruch 1, worin
R¹ für Wasserstoff, Methyl, Ethyl, n- oder i-Propyl steht,
R² für Wasserstoff, Methyl, Ethyl, n-Propyl, i-Propyl oder n-Butyl, Cyclopropyl, Cyclopentyl, Cyclohexyl oder für einen Rest -CH₂R³ steht,
R³ für C₂-C₅-Alkenyl, C₂-C₅-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, durch ein oder zwei (bevorzugt einen) Substituenten aus der Gruppe C₁-C₃-Alkyl, C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₁-C₃-Alkoxy, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Halogen, Cyan und Nitro substituiertes 5-Thiazolyl; oder durch ein oder zwei (bevorzugt einen) Reste aus der Gruppe C₁-C₃-Halogenalkyl mit 1 bis 7 Halogenatomen, Cyclopropyl, Halogencyclopropyl mit 1 bis 3 Halogenatomen, C₂-C₃-Alkenyl, C₂-C₃-Alkinyl, C₂-C₃-Halogenalkenyl und C₂-C₃-Halogenalkinyl mit jeweils 1 bis 4 Halogenatomen, C₁-C₃-Halogenalkoxy mit 1 bis 7 Halogenatomen, C₁-C₃-Alkylthio, C₁-C₃-Halogenalkylthio mit 1 bis 7 Halogenatomen, Allyloxy, Propargyloxy, Allylthio, Propargylthio, Halogenallyloxy und Halogenallylthio mit jeweils 1 bis 3 Halogenatomen, Cyano, Nitro, C₁-C₃-Alkyl, C₁-C₃-Alkoxy und Halogen substituiertes 3-Pyridyl steht,
R⁴ für C₁-C₁₀-Alkyl, C₃-C₆-Cycloalkyl, durch 1 bis 12 Reste aus der Gruppe Halogen, Hydroxy, C₁-C₄-Alkoxy, C₁-C₄-Halogenalkoxy mit 1 bis 9 Halogenatomen, Di-( C₁-C₄-Alkyl)-amino und C₁-C₅-Alkoxycarbonyl substituiertes C₁-C₁₀-Alkyl, durch 1 bis 4 Reste aus der Reihe C₁-C₄-Alkyl und Halogen substituiertes C₃-C₆-Cycloalkyl, Phenyl, Benzyl, oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro und Cyano substituiertes Phenyl oder Benzyl oder Heterocyclylmethyl, wobei Heterocyclyl für einen ungesättigten oder gesättigten 5- oder 6-gliedrigen Heterocyclus mit einem oder zwei (bevorzugt einem) Heteroatomen aus der Reihe Stickstoff, Sauerstoff und Schwefel wie z.B. Furan, Tetrahydrofuran, Thiophen oder Pyridin steht,
Het für einen unsubstituierten oder einfach oder zweifach (bevorzugt einfach) substituierten heterocyclischen Rest aus der Reihe 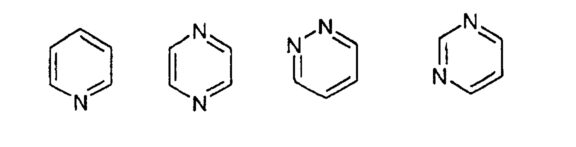 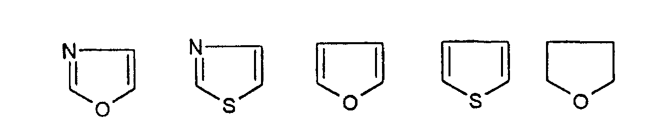 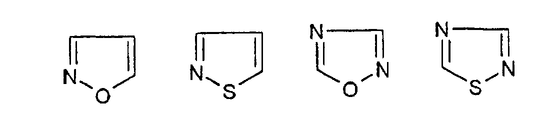 wobei die Substituenten aus der Reihe Fluor, Chlor, Brom, Methyl, Ethyl, Methoxy und Ethoxy ausgewählt sind, steht, in Gegenwart eines Verdünnungsmittels mit Harnstoff umsetzt.

3. Verfahren gemäß Anspruch 1, **dadurch gekennzeichnet, daß** man Verbindungen der Formel (II) gemäß Anspruch 1, worin
R¹ für Wasserstoff oder Methyl steht,
R² Wasserstoff, Methyl, Ethyl, n-Propyl, i-Propyl, n-Butyl, Cyclopropyl, Cyclopentyl, Cyclohexyl oder für einen Rest -CH₂R³ steht,
R³ für C₂-C₃-Alkenyl, C₂-C₃-Alkinyl, Phenyl, Cyanophenyl, Nitrophenyl, Halogenphenyl mit 1 bis 3 Halogenatomen, durch C₁-C₃-Alkyl, C₁-C₃-Halogenalkyl mit 1 bis 7 Halogenatomen, C₁-C₃-Alkoxy oder C₁-C₃-Halogenalkoxy mit 1 bis 7 Halogenatomen substituiertes Phenyl, 3-Pyridyl, 5-Thiazolyl, jeweils durch ein oder zwei (bevorzugt einen) Substituenten aus der Gruppe C₁-C₃-Alkyl, C₁-C₃-Halogenalkyl mit 1 bis 7 Halogenatomen, C₁-C₃-Alkoxy, C₁-C₃-Halogenalkoxy mit 1 bis 7 Halogenatomen, C₁-C₃-Alkylthio, C₁-C₃-Halogenalkylthio mit 1 bis 7 Halogenatomen, Halogen, Cyano und Nitro substituiertes 5-Thiazolyl oder 3-Pyridyl steht,
R⁴ für C₁-C₁₀-Alkyl, C₃-C₆-Cycloalkyl, durch 1 bis 6 Reste aus der Gruppe Halogen, C₁-C₄-Alkoxy und C₁-C₄-Halogenalkoxy mit jeweils 1 bis 9 Halogenatomen substituiertes C₁-C₈-Alkyl, durch 1 oder 2 Reste aus der Reihe Methyl, Ethyl, Fluor oder Chlor substituiertes C₃-C₆-Cycloalkyl, Phenyl, Benzyl, oder jeweils durch 1 bis 3 Ringsubstituenten aus der Gruppe Halogen, C₁-C₄-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, C₁-C₄-Alkylthio, Nitro und Cyano substituiertes Phenyl oder Benzyl, oder Heterocyclylmethyl steht, wobei Heterocyclyl für einen ungesättigten oder gesättigten 5- oder 6-gliedrigen Heterocyclus mit einem Heteroatom aus der Reihe Stickstoff, Sauerstoff und Schwefel wie z.B. Furan, Tetrahydrofuran, Thiophen oder Pyridin steht,
Het für jeweils unsubstituiertes oder einfach oder zweifach (insbesondere einfach) substituiertes Thiazolyl, Pyridyl oder Tetrahydrofuranyl, wobei die Substituenten aus der Reihe Fluor, Chlor, Methyl und Methoxy (insbesondere Chlor) ausgewählt sind, steht, in Gegenwart eines Verdünnungsmittels mit Harnstoff umsetzt.

4. Verfahren gemäß Anspruch 1, **dadurch gekennzeichnet, daß** man eine Verbindung der Formel (II), gemäß Anspruch 1, worin
R¹ für Wasserstoff oder Methyl, hervorgehoben für Wasserstoff steht,
R² für Wasserstoff, Methyl, Ethyl, n-Propyl, Cyclopropyl, Cyclopentyl, Allyl oder Propargyl steht,
R⁴ für Methyl, Ethyl, n-Propyl, Cyclopropyl, Cyclopentyl, Cyclohexyl, Phenyl, Benzyl oder Furfurylmethyl steht,
Het für einen der Reste 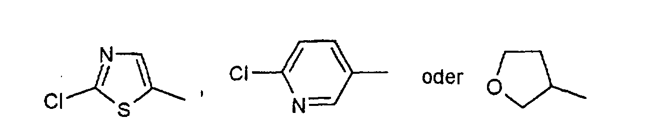 steht, in Gegenwart eines Verdünnungsmittels mit Harnstoff umsetzt.

5. Verbindung der Formel

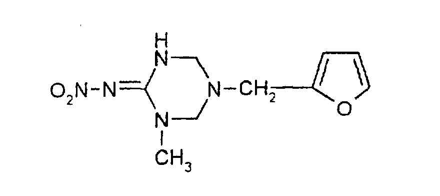

6. Verbindung der Formel

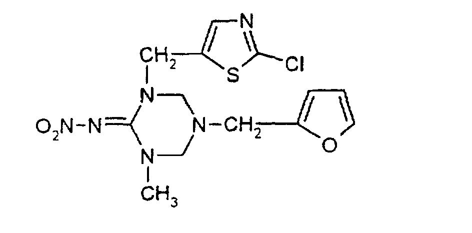

## Claims

1. Process for the preparation of compounds of the formula (I) 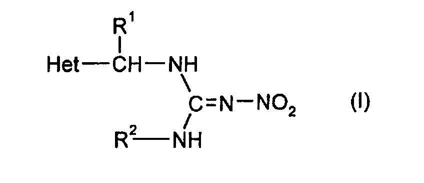 in which
R¹ is hydrogen or C₁-C₄-alkyl,
R² is hydrogen, C₁-C₆-alkyl, C₃-C₆-cycloalkyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl substituted by one to two (preferably one) substituents from the group consisting of C₁-C₃-alkyl, C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₁-C₃-alkoxy, C₂-C₃-halogenoalkenyl and C₂-C₃-halogenoalkinyl having in each case from 1 to 4 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy and halogenoallylthio having in each case from 1 to 3 halogen atoms, halogen, cyano and nitro; or 3-pyridyl substituted by one to four (preferably one or two) radicals from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃-halogenoalkenyl and C₂-C₃-halogenoalkinyl having in each case from 1 to 4 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy and halogenoallylthio having in each case from 1 to 3 halogen atoms, cyano, nitro, C₁-C₃-alkyl, C₁-C₃-alkoxy and halogen,
Het is an unsubstituted or substituted aromatic or non-aromatic, monocyclic or bicyclic heterocyclic radical which may include one to two substituents from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃-halogenoalkenyl and C₂-C₃-halogenoalkinyl having from 1 to 4 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy, halogenoallylthio, cyano, nitro, C₁-C₃-alkyl, C₁-C₃-alkoxy and halogen,
**characterized in that** a compound of the formula (II) 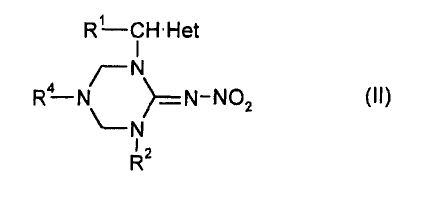 in which
R¹, R² and Het are as defined above, and
R⁴ is C₁-C₁₀-alkyl, C₃-C₆-cycloalkyl, phenyl, benzyl or heterocyclylmethyl, each of which may be unsubstituted or substituted, where heterocyclyl is an unsaturated or saturated 5- or 6-membered heterocycle containing one or two (preferably one) heteroatoms from the series nitrogen, oxygen and sulphur, such as, for example, furan, tetrahydrofuran, thiophene or pyridine,
is reacted with urea in the presence of a diluent.

2. Process according to Claim 1, **characterized in that** a compound of the formula (II) according to Claim 1, in which
R¹ is hydrogen, methyl, ethyl, n- or i-propyl,
R² is hydrogen, methyl, ethyl, n-propyl, i-propyl or n-butyl, cyclopropyl, cyclopentyl, cyclohexyl or a radical -CH₂R³,
R³ is C₂-C₅-alkenyl, C₂-C₅-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl substituted by one to two (preferably one) substituents from the group consisting of C₁-C₃-alkyl, C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₁-C₃-alkoxy, C₂-C₃-halogenoalkenyl and C₂-C₃-halogenoalkinyl having in each case from 1 to 4 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy and halogenoallylthio having in each case from 1 to 3 halogen atoms, halogen, cyano and nitro; or is 3-pyridyl substituted by one to two (preferably one) radicals from the group consisting of C₁-C₃-halogenoalkyl having from 1 to 7 halogen atoms, cyclopropyl, halogenocyclopropyl having from 1 to 3 halogen atoms, C₂-C₃-alkenyl, C₂-C₃-alkinyl, C₂-C₃-halogenoalkenyl and C₂-C₃-halogenoalkinyl having in each case from 1 to 4 halogen atoms, C₁-C₃-halogenoalkoxy having from 1 to 7 halogen atoms, C₁-C₃-alkylthio, C₁-C₃-halogenoalkylthio having from 1 to 7 halogen atoms, allyloxy, propargyloxy, allylthio, propargylthio, halogenoallyloxy and halogenoallylthio having in each case from 1 to 3 halogen atoms, cyano, nitro, C₁-C₃-alkyl, C₁-C₃-alkoxy and halogen,
R⁴ is C₁-C₁₀-alkyl, C₃-C₆-cycloalkyl, C₁-C₁₀-alkyl substituted by from 1 to 12 radicals from the group consisting of halogen, hydroxyl, C₁-C₄-alkoxy, C₁-C₄-halogenoalkoxy having from 1 to 9 halogen atoms, di-(C₁-C₄-alkyl)-amino and C₁-C₅-alkoxycarbonyl, C₃-C₆-cycloalkyl substituted by from 1 to 4 radicals from the series C₁-C₄-alkyl and halogen, phenyl, benzyl, or phenyl or benzyl in each case substituted by from 1 to 3 ring substituents from the group consisting of halogen, C₁-C₄-alkyl, C₁-C₄-halogenoalkyl having from 1 to 9 halogen atoms, C₁-C₄-alkoxy, C₁-C₄-halogenoalkoxy having from 1 to 9 halogen atoms, C₁-C₄-alkylthio, nitro and cyano, or heterocyclylmethyl, where heterocyclyl is an unsaturated or saturated 5- or 6-membered heterocycle having one or two (preferably one) heteroatoms from the series nitrogen, oxygen and sulphur, such as, for example, furan, tetrahydrofuran, thiophene or pyridine,
Het is an unsubstituted or mono- or disubstituted (preferably monosubstituted) heterocyclic radical from the series 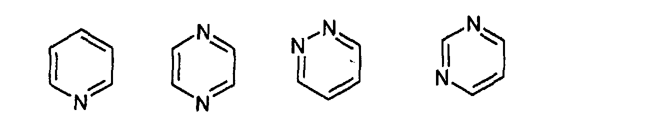 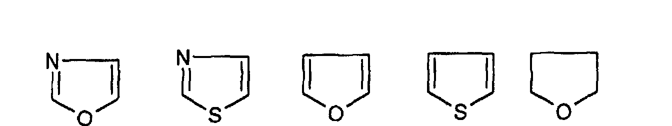 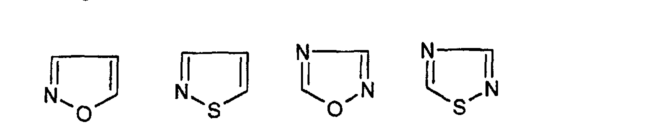
the substituents being chosen from the series fluorine, chlorine, bromine, methyl, ethyl, methoxy and ethoxy, is reacted with urea in the presence of a diluent.

3. Process according to Claim 1, **characterized in that** compounds of the formula (II) according to Claim 1, in which
R¹ is hydrogen or methyl,
R² is hydrogen, methyl, ethyl, n-propyl, i-propyl, n-butyl, cyclopropyl, cyclopentyl, cyclohexyl or a radical -CH₂R³,
R³ is C₂-C₃-alkenyl, C₂-C₃-alkinyl, phenyl, cyanophenyl, nitrophenyl, halogenophenyl having from 1 to 3 halogen atoms, phenyl substituted by C₁-C₃-alkyl, C₁-C₃-halogenoalkyl having from 1 to 7 halogen atoms, C₁-C₃-alkoxy or C₁-C₃-halogenoalkoxy having from 1 to 7 halogen atoms, 3-pyridyl, 5-thiazolyl, 5-thiazolyl or 3-pyridyl each substituted by one or two (preferably one) substituents from the group consisting of C₁-C₃-alkyl, C₁-C₃-halogenoalkyl having from 1 to 7 halogen atoms, C₁-C₃-alkoxy, C₁-C₃-halogenoalkoxy having from 1 to 7 halogen atoms, C₁-C₃-alkylthio, C₁-C₃-halogenoalkylthio having from 1 to 7 halogen atoms, halogen, cyano and nitro,
R⁴ is C₁-C₁₀-alkyl, C₃-C₆-cycloalkyl, C₁-C₈-alkyl, substituted by from 1 to 6 radicals from the group consisting of halogen, C₁-C₄-alkoxy and C₁-C₄-halogenoalkoxy having in each case from 1 to 9 halogen atoms, C₃-C₆-cycloalkyl substituted by 1 or 2 radicals from the series methyl, ethyl, fluorine or chlorine, phenyl, benzyl, or phenyl or benzyl each substituted by from 1 to 3 ring substituents from the group consisting of halogen, C₁-C₄-alkyl, C₁-C₄-halogenoalkyl having from 1 to 9 halogen atoms, C₁-C₄-alkoxy, C₁-C₄-halogenoalkoxy having from 1 to 9 halogen atoms, C₁-C₄-alkylthio, nitro and cyano, or heterocyclyl-methyl, where heterocyclyl is an unsaturated or saturated 5- or 6-membered heterocycle containing one heteroatom from the series nitrogen, oxygen and sulphur, such as, for example, furan, tetrahydrofuran, thiophene or pyridine,
Het is thiazolyl, pyridyl or tetrahydrofuranyl, each of which may be unsubstituted or mono-or disubstituted (in particular monosubstituted), the substituents being chosen from the series fluorine, chlorine, methyl and methoxy (in particular chlorine), are reacted with urea in the presence of a diluent.

4. Process according to Claim 1, **characterized in that** a compound of the formula (II) according to Claim 1, in which
R¹ is hydrogen or methyl, especially hydrogen,
R² is hydrogen, methyl, ethyl, n-propyl, cyclopropyl, cyclopentyl, allyl or propargyl,
R⁴ is methyl, ethyl, n-propyl, cyclopropyl, cyclopentyl, cyclohexyl, phenyl, benzyl or furfurylmethyl,
Het is one of the radicals 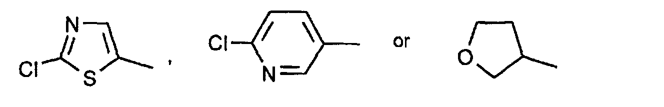 is reacted with urea in the presence of a diluent.

5. Compound of the formula

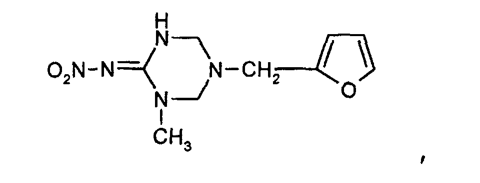

6. Compound of the formula

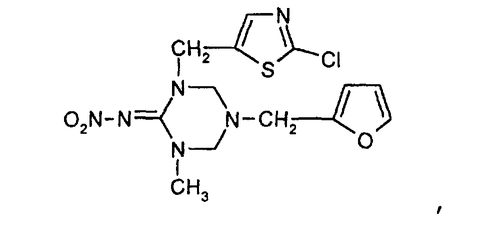

## Revendications

1. Procédé de préparation de composés de formule (I) : 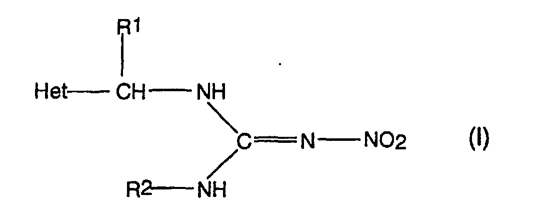 dans laquelle :
R¹ représente l'atome d'hydrogène ou un radical alcoyle en C₁-C₄ ;
R² représente l'atome d'hydrogène, un radical alcoyle en C₁-C₆, un radical cycloalcoyle en C₃-C₆ ou un reste -CH₂R³ ;
R³ représente un radical alcényle en C₂-C₅, un radical alcynyle en C₂-C₅, un radical phényle, un radical cyanophényle, un radical nitrophényle, un radical halogénophényle ayant 1 à 3 atomes d'halogène, un radical phényle substitué par un radical alcoyle en C₁-C₃, par un radical halogénoalcoyle en C₁-C₃ ayant 1 à 7 atomes d'halogène, par un radical alcoxy en C₁-C₃, par un radical halogénoalcoxy en C₁-C₃ ayant 1 à 7 atomes d'halogène, ou représente un radical 3-pyridyle, un radical 5-thiazolyle, un radical 5-thiazolyle substitué par un ou deux substituants (de préférence, un substituant) parmi le groupe des radicaux alcoyle en C₁-C₃, halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, alcoxy en C₁-C₃, halogénoalcényle en C₂-C₃ et halogénoalcynyle en C₂-C₃ ayant chaque fois 1 à 4 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy et halogénoallylthio ayant chaque fois 1 à 3 atomes d'halogène, un atome d'halogène, les radicaux cyano et nitro ; ou représente un radical 3-pyridyle substitué par un à quatre (de préférence, un ou deux) restes parmi le groupe des radicaux halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, halogénoalcényle en C₂-C₃ et halogénoalcynyle en C₂-C₃ ayant chaque fois 1 à 4 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy et halogénoallylthio ayant chaque fois 1 à 3 atomes d'halogène, cyano, nitro, alcoyle en C₁C₃, alcoxy en C₁-C₃, les atomes d'halogène ;
Het représente un reste hétérocyclique aromatique ou non aromatique, monocyclique ou bicyclique, non substitué ou substitué, qui peut contenir un ou deux substituants parmi le groupe des radicaux halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, halogénoalcényle en C₂-C₃ et halogénoalcynyle en C₂-C₃ ayant 1 à 4 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy, halogénoallylthio, cyano, nitro, alcoyle en C₁-C₃, alcoxy en C₁-C₃ et les atomes d'halogène,
**caractérisé en ce que** l'on fait réagir un composé de formule (II) : 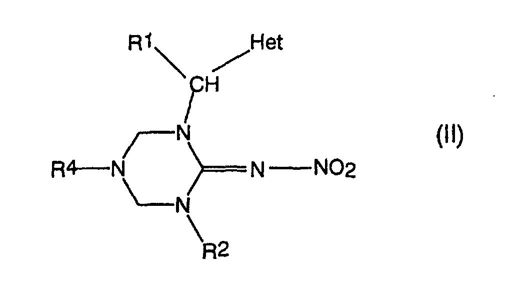 où
R¹, R² et Het ont les significations indiquées ci-dessus, et
R⁴ représente un radical alcoyle en C₁-C₁₀, un radical cycloalcoyle en C₃-C₆, un radical phényle, un radical benzyle ou un radical hétérocyclylméthyle, chaque fois substitués ou non substitués, où le radical hétérocyclyle représente un hétérocycle à 5 ou 6 membres, insaturé ou saturé, avec un ou deux (de préférence un) hétéroatomes de la série des atomes d'azote, d'oxygène et de soufre, comme par exemple le furanne, le tétrahydrofuranne, le thiophène ou la pyridine,
en présence d'un agent de dilution, avec de l'urée.

2. Procédé suivant la revendication 1, **caractérisé en ce que** l'on fait réagir avec de l'urée en présence d'un agent de dilution, un composé de formule (II) suivant la revendication 1, où :
R¹ représente l'atome d'hydrogène, les radicaux méthyle, éthyle, n- ou i-propyle ;
R² représente l'atome d'hydrogène, les radicaux méthyle, éthyle, n-propyle, i-propyle ou n-butyle, cyclopropyle, cyclopentyle, cyclohexyle ou un reste - CH₂R³ ;
R³ représente un radical alcényle en C₂-C₅, un radical alcynyle en C₂-C₅, un radical phényle, un radical cyanophényle, un radical nitrophényle, un radical halogénophényle ayant 1 à 3 atomes d'halogène, un radical phényle substitué par un radical alcoyle en C₁-C₃, par un radical halogénoalcoyle en C₁-C₃ ayant 1 à 7 atomes d'halogène, par un radical alcoxy en C₁-C₃ ou par un radical halogénoalcoxy en C₁-C₃ ayant 1 à 7 atomes d'halogène, ou représente un radical 3-pyridyle, un radical 5-thiazolyle,un radical 5-thiazolyle substitué par un ou deux substituants (de préférence, un substituant) parmi le groupe des radicaux alcoyle en C₁-C₃, halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, alcoxy en C₁-C₃, halogénoalcényle en C₂-C₃ et halogénoalcynyle en C₂-C₃ ayant chaque fois 1 à 4 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy et halogénoallylthio ayant chaque fois 1 à 3 atomes d'halogène, un atome d'halogène, les radicaux cyano et nitro ; ou représente un radical 3-pyridyle substitué par un ou deux restes (de préférence, un reste ) parmi le groupe des radicaux halogénoalcoyle en C₁-C₃ ayant 1 à 7 atomes d'halogène, cyclopropyle, halogénocyclopropyle ayant 1 à 3 atomes d'halogène, alcényle en C₂-C₃, alcynyle en C₂-C₃, halogénoalcényle en C₂-C₃ et halogénoalcynyle en C₂-C₃ ayant chaque fois 1 à 4 atomes d'halogène, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, allyloxy, propargyloxy, allylthio, propargylthio, halogénoallyloxy et halogénoallylthio ayant chaque fois 1 à 3 atomes d'halogène, cyano, nitro, alcoyle en C₁C₃, alcoxy en C₁-C₃ et les atomes d'halogène ;
R⁴ représente un radical alcoyle en C₁-C₁₀, un radical cycloalcoyle en C₃-C₆, un radical alcoyle en C₁C₁₀ substitué par 1 à 12 restes parmi le groupe des atomes d'halogène, des radicaux hydroxy, alcoxy en C₁-C₄, halogénoalcoxy en C₁-C₄ ayant 1 à 9 atomes d'halogène, di(alcoyl en C₁-C₄)amino et alcoxycarbonyle en C₁-C₅, cycloalcoyle en C₃-C₆ substitué par 1 à 4 restes de la série des radicaux alcoyle en C₁-C₄ et des atomes d'halogène, ou représente un radical phényle, un radical benzyle ou les radicaux phényle ou benzyle substitués chaque fois par 1 à 3 substituants sur le cycle, parmi le groupe des atomes d'halogène, alcoyle en C₁-C₄, halogénoalcoyle en C₁-C₄ ayant 1 à 9 atomes d'halogène, alcoxy en C₁-C₄, halogénoalcoxy en C₁-C₄ ayant 1 à 9 atomes d'halogène, alcoylthio en C₁C₄, nitro et cyano ; ou représente un radical hétérocyclylméthyle, où le radical hétérocyclyle représente un hétérocycle à 5 ou 6 membres, insaturé ou saturé, avec un ou deux hétéroatomes (de préférence un hétéroatome) de la série des atomes d'azote, d'oxygène et de soufre, comme par exemple le furanne, le tétrahydrofuranne, le thiophène ou la pyridine ;
Het représente de préférence, un reste hétérocyclique non substitué ou substitué une ou deux fois (de préférence, une fois) de la série : 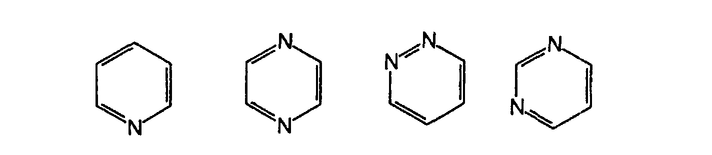 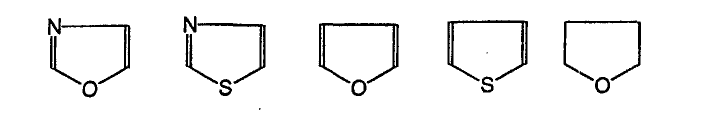 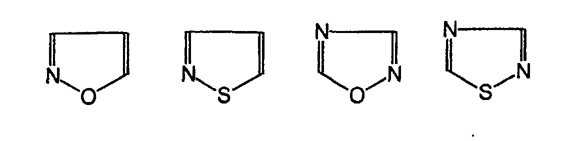
où les substituants sont choisis parmi le série des atomes de fluor, de chlore, de brome, les radicaux méthyle, éthyle, méthoxy et éthoxy.

3. Procédé suivant la revendication 1, **caractérisé en ce que** l'on fait réagir avec de l'urée en présence d'un agent de dilution, des composés de formule (II) suivant la revendication 1, où :
R¹ représente l'atome d'hydrogène ou le radical méthyle ;
R² représente l'atome d'hydrogène, les radicaux méthyle, éthyle, n-propyle, i-propyle, n-butyle, cyclopropyle, cyclopentyle, cyclohexyle ou un reste - CH₂R³ ;
R³ représente un radical alcényle en C₂-C₃, un radical alcynyle en C₂-C₃, un radical phényle, un radical cyanophényle, un radical nitrophényle, un radical halogénophényle ayant 1 à 3 atomes d'halogène, un radical phényle substitué par un radical alcoyle en C₁-C₃, par un radical halogénoalcoyle en C₁-C₃ ayant 1 à 7 atomes d'halogène, par un radical alcoxy en C₁-C₃ ou par un radical halogénoalcoxy en C₁-C₃ ayant 1 à 7 atomes d'halogène, ou représente un radical 3-pyridyle, un radical 5-thiazolyle, ou les radicaux 5-thiazolyle ou 3-pyridyle chaque fois substitués par un ou deux substituants (de préférence, un substituant) parmi le groupe des radicaux alcoyle en C₁-C₃, halogénoalcoyle en C₁-C₃ ayant 1 à 7 atomes d'halogène, alcoxy en C₁-C₃, halogénoalcoxy en C₁-C₃ ayant 1 à 7 atomes d'halogène, alcoylthio en C₁-C₃, halogénoalcoylthio en C₁-C₃ ayant 1 à 7 atomes d'halogène, les atomes d'halogène, les radicaux cyano et nitro ;
R⁴ représente un radical alcoyle en C₁-C₁₀, un radical cycloalcoyle en C₃-C₆, un radical alcoyle en C₁-C₈ substitué par 1 à 6 restes parmi le groupe des atomes d'halogène, des radicaux alcoxy en C₁-C₄ et halogénoalcoxy en C₁-C₄ ayant 1 à 9 atomes d'halogène, ou représente un radical cycloalcoyle en C₃-C₆ substitué par 1 ou 2 restes de la série comportant les radicaux méthyle, éthyle, les atomes de fluor ou de chlore, ou représente un radical phényle, un radical benzyle ou les radicaux phényle ou benzyle substitués chaque fois par 1 à 3 substituants sur le cycle, parmi le groupe des atomes d'halogène, des radicaux alcoyle en C₁-C₄, halogénoalcoyle en C₁-C₄ ayant 1 à 9 atomes d'halogène, alcoxy en C₁-C₄, halogénoalcoxy en C₁-C₄ ayant 1 à 9 atomes d'halogène, alcoylthio en C₁-C₄, nitro et cyano, ou représente un radical hétérocyclylméthyle, où le radical hétérocyclyle représente un hétérocycle à 5 ou 6 membres, insaturé ou saturé, avec un hétéroatome de la série des atomes d'azote, d'oxygène et de soufre, comme par exemple le furanne, le tétrahydrofuranne, le thiophène ou la pyridine ;
Het représente un radical thiazolyle, un radical pyridyle ou un radical tétrahydrofurannyle, chaque fois non substitué ou substitué une ou deux fois (en particulier, une fois), où les substituants sont choisis parmi la série des atomes de fluor, de chlore, les radicaux méthyle et méthoxy (en particulier, l'atome de chlore).

4. Procédé suivant la revendication 1, **caractérisé en ce que** l'on fait réagir avec de l'urée en présence d'un agent de dilution, des composés de formule (II) suivant la revendication 1, où :
R¹ représente l'atome d'hydrogène ou le radical méthyle ; spécifiquement l'atome d'hydrogène ;
R² représente l'atome d'hydrogène, les radicaux méthyle, éthyle, n-propyle, cyclopropyle, cyclopentyle, allyle ou propargyle ;
R⁴ représente les radicaux méthyle, éthyle, n-propyle, cyclopropyle, cyclopentyle, cyclohexyle, phényle, benzyle ou furfurylméthyle ;
Het représente l'un des restes :

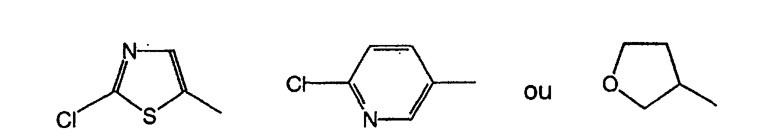

5. Composé de formule :

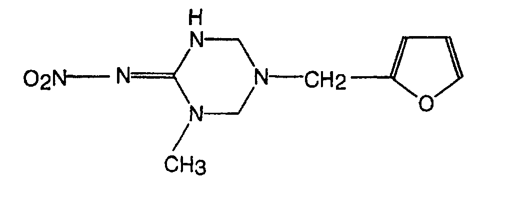

6. Composé de formule :
